# EUROPEAN PATENT APPLICATION

(11) **EP 4 623 904 A1**
(43) Date of publication of application: **01.10.2025**
(21) Application number: 23894871.5
(22) Date of filing: 03.11.2023
(51) Int. Cl.: A61K 9/16

(54) **BIODEGRADABLE POLYMER DISPERSION AND PREPARATION METHOD THEREFOR**

(30) Priority: 23.11.2022 KR 20220158247
(71) Applicant: Vaim Co., Ltd., Daejeon 34016 (KR)
(72) Inventor: KIM, Gun Poong, Nonsan-si Chungcheongnam-do 33027 (KR); SEO, Suk Bae, Seoul 06732 (KR)
(74) Representative: Kraus & Lederer PartGmbB
(86) International application number: PCT/KR2023/017530
(87) International publication number: WO 2024/111942

(57) **Abstract**

The present invention relates to a biodegradable polymer dispersion comprising: a lactic acid-based polymer, a block copolymer including a lactic acid-based polymer and polyethylene glycol, and a high molecular weight hyaluronic acid, and a method for preparing the same. The polymer dispersion allows effective support of functional active ingredients, exhibits excellent physiological activity on its own upon penetration into the skin, and has significantly improved dispersion stability to provide an excellent delivery effect to tissues.

## Description

### [Technical Field]

The present invention relates to a biodegradable polymer dispersion and a method for preparing the same.

### [Background Art]

The skin is composed of epidermis, dermis, and subcutaneous tissue. The dermis is a layer between the epidermis and subcutaneous tissue, and has blood vessels, collagen, elastin fibers, pores, arrector pili, sebaceous glands, sweat glands, various sensory nerves, fibroblasts, macrophages, and the like which do not exist in the epidermis, and is a part which is the most directly related to skin aging. 80% of the dermal layer is composed of collagen which is sensitive to UV exposure and is prone to aging by external stimuli.

In order to improve skin state through percutaneous absorption of an active ingredient (active component), the component should penetrate a skin barrier layer present in the epidermis. However, since most of the active ingredients are not delivered to deep tissues of the skin without penetrating the skin barrier layer, use of a chemical absorption promoter, a method for physically forming micropores in the skin barrier layer, a method for delivering the active ingredient by iontophoresis, or the like has been considered, in order to improve percutaneous absorption of the active ingredient.

Meanwhile, in order to improve penetration and permeation of the active ingredient which is difficult to be absorbed into the skin, a pharmaceutical approach has been widely studied, and in particular, development of a drug delivery system such as colloids or nanoparticles or a formulation approach has been intensively studied.

As an active ingredient delivery system based on the drug delivery system, nanoparticles such as liposome, cationic polymers, quantum dots, magnetic particles, or gold nanoparticles have been studied, and the delivery system was designed to promote absorption into cells. Liposome has a benefit of easy design so that it has a targeting performance as a physical self-assembly, but has poor colloid stability of a delivery system, and is mainly dependent on delivery of an active ingredient by diffusion in the skin barrier layer. In addition, when cationic polymers, quantum dots, gold nanoparticles, and the like are used, there is some cytotoxicity, effective delivery of an active ingredient is not easy, and the delivery system does not have biodegradability.

In order to solve the problem, recently, a system of controlling delivery or release of an active ingredient by enclosing the active ingredient in a carrier using a biodegradable polymer has been studied. However, the method is mainly appropriate for enclosing an active ingredient having a low molecular weight, and when a high molecular weight active ingredient is enclosed, the active ingredient is difficult to control the release and is initially released in excess.

Thus, development of an efficient formulation which suppresses excessive initial release of an active ingredient, may continuously release the active ingredient, and may adjust the release rate of the active ingredient as desired is required.

### [Related Art Document]

### [Patent Document]

Korean Patent Laid-Open Publication No. 10-2019-0095088 (August 19, 2019)

### [Disclosure]

### [Technical Problem]

An object of the present invention is to provide a biodegradable polymer dispersion which may effectively deliver an active ingredient into a cell, and a method for preparing the same, and specifically provide a biodegradable polymer dispersion which penetrates the skin and shows physiological activity in itself to be effective for improving a skin state and a method for preparing the same.

Another object of the present invention is to provide a pharmaceutical composition including a biodegradable polymer dispersion, which may effectively deliver an active ingredient into a deep layer of the skin, by including the active ingredient inside particles including a lactic acid-based polymer.

### [Technical Solution]

In one general aspect, a biodegradable polymer dispersion includes: a lactic acid-based polymer; a block copolymer including a lactic acid-based polymer and polyethylene glycol; and a high molecular weight hyaluronic acid having a weight average molecular weight of 500,000 g/mol or more.

In an exemplary embodiment of the present invention, a weight ratio between the hyaluronic acid mixture and the lactic acid-based polymer may be 1.5:1 to 5:1.

In an exemplary embodiment of the present invention, the hyaluronic acid may further include one or more of a hyaluronic acid oligomer having a weight average molecular weight of less than 6,000 g/mol and an ammonium-substituted hyaluronic acid.

In an exemplary embodiment of the present invention, the hyaluronic acid oligomer may be included at 5 to 20 wt% with respect to the total weight of the hyaluronic acid.

In an exemplary embodiment of the present invention, the biodegradable polymer dispersion may have an average particle size of 0.01 to 30 µm.

In an exemplary embodiment of the present invention, the lactic acid-based polymer may have a weight average molecular weight of 10,000 to 1,000,000 g/mol.

In an exemplary embodiment of the present invention, the block copolymer including the lactic acid-based polymer and polyethylene glycol may have a weight average molecular weight of 2,000 to 60,000 g/mol.

In an exemplary embodiment of the present invention, a weight ratio between the lactic acid-based polymer and the block copolymer including the lactic acid-based polymer and polyethylene glycol may be 10:1 to 1:1.

In an exemplary embodiment of the present invention, the block copolymer including the lactic acid-based polymer and polyethylene glycol may be positioned on the surface of the particles including the lactic acid-based polymer.

In another general aspect, a pharmaceutical composition includes the biodegradable polymer dispersion described above.

In still another general aspect, a method for preparing a biodegradable polymer dispersion includes: dissolving a block copolymer including a lactic acid-based polymer and polyethylene glycol and a lactic acid-based polymer in a first organic solvent to prepare a biodegradable polymer solution; mixing a high molecular weight hyaluronic acid having a weight average molecular weight of 500,000 g/mol or more and water to prepare a hyaluronic acid aqueous solution; mixing the hyaluronic acid aqueous solution with the biodegradable polymer solution to prepare a first dispersion liquid; and removing the first organic solvent from the first dispersion liquid to obtain a first emulsion.

In an exemplary embodiment of the present invention, the organic solvent may be one or a mixture of two or more selected from the group consisting of acetone, ethanol, methylene chloride, chloroform, ethyl acetate, tetrahydrofuran, N,N-dimethylformamide, and N,N-dimethylacetamide.

In an exemplary embodiment of the present invention, the hyaluronic acid aqueous solution may be further mixed with a high molecular weight hyaluronic acid having a weight average molecular weight of 500,000 g/mol or more; and one or more of a hyaluronic acid oligomer having a weight average molecular weight of less than 6,000 g/mol and an ammonium-substituted hyaluronic acid.

In an exemplary embodiment of the present invention, the method may further include: redispersing the first emulsion in a second organic solvent to prepare a second dispersion liquid; and removing the second organic solvent from the second dispersion liquid.

In an exemplary embodiment of the present invention, a ratio of nanoparticles having an average particle diameter of 100 to 1000 nm in the biodegradable polymer dispersion may be 20% or more.

### [Advantageous Effects]

The biodegradable polymer dispersion according to the present invention includes lactic acid-based polymer particles in which a block copolymer (PEG-PLA) including a lactic acid-based polymer and polyethylene glycol is positioned on the surface of a water phase containing a hyaluronic acid mixture, in a dispersed form, thereby having excellent dispersion stability, allowing control of the particle size of the lactic acid-based polymer, and is capable of penetrating the skin independently to exert physiological activity.

In addition, the biodegradable polymer dispersion according to the present invention includes an active ingredient, thereby effectively delivering the active ingredient through the skin or other barriers. Specifically, the active ingredient may include both hydrophilic and hydrophobic active ingredients, thereby having excellent physiological activity and implementing a treatment or improvement effect. Furthermore, the biodegradable polymer dispersion further includes an active ingredient inside the lactic acid-based polymer particles, thereby controlling continuous release of the active ingredient.

### [Description of Drawings]

FIG. 1 is graphs showing expression levels of a dermal fibroblast precursor Lrig1 and a papillary dermal fibroblast precursor Blimp1, when adipose-derived stem cells were treated with the biodegradable polymer dispersion according to Example 1 of the present invention and the polymers according to Comparative Examples 1 and 2.
FIG. 2 is graphs showing expression levels of reticular dermis/ subcutaneous fibroblast precursor Dlk1 and papillary dermal fibroblasts FSP1, when adipose-derived stem cells were treated with the biodegradable polymer dispersion according to Example 1 of the present invention and the polymers according to Comparative Examples 1 and 2.
FIG. 3 is graphs showing mRNA expression levels of growth factors bFGF, VEGF, and HGF, when adipose-derived stem cells were treated with the biodegradable polymer dispersion according to Example 1 of the present invention and the polymers according to Comparative Examples 1 and 2.
FIG. 4 is graphs showing mRNA expression levels of growth factors TGF-β1, TGF-β2, and TGF-β3, when adipose-derived stem cells were treated with the biodegradable polymer dispersion according to Example 1 of the present invention and the polymers according to Comparative Examples 1 and 2.
FIG. 5 is a graph showing changes in measured moisture values (Δskin moisture), 8 weeks after the biodegradable polymer dispersion according to Example 1 and the polymers according to Comparative Examples 1 and 2 were injected into a B6 mouse, regarding Experimental Example 3 of the present invention.
FIG. 6 is graph showing elasticity values measured 8 weeks after the biodegradable polymer dispersion according to Example 1 and the polymers according to Comparative Examples 1 and 2 were injected into a B6 mouse, regarding Experimental Example 3 of the present invention.
FIG. 7 is microscope images for confirming changes in a basement membrane, 8 weeks after the biodegradable polymer dispersion according to Example 1 and the polymers according to Comparative Examples 1 and 2 were injected into a B6 mouse, regarding Experimental Example 3 of the present invention.
FIG. 8 is a graph showing changes in a basement membrane, 8 weeks after the biodegradable polymer dispersion according to Example 1 and the polymers according to Comparative Examples 1 and 2 were injected into a B6 mouse, regarding Experimental Example 3 of the present invention.
FIG. 9 is microscope images for confirming changes in collagen fibers, 8 weeks after the biodegradable polymer dispersion according to Example 1 and the polymers according to Comparative Examples 1 and 2 were injected into a B6 mouse, regarding Experimental Example 3 of the present invention.
FIG. 10 is a graph showing changes in collagen fibers, 8 weeks after the biodegradable polymer dispersion according to Example 1 and the polymers according to Comparative Examples 1 and 2 were injected into a B6 mouse, regarding Experimental Example 3 of the present invention.
FIG. 11 is microscope images for confirming changes in elastin fibers, 8 weeks after the biodegradable polymer dispersion according to Example 1 and the polymers according to Comparative Examples 1 and 2 were injected into a B6 mouse, regarding Experimental Example 3 of the present invention.
FIG. 12 is a graph showing changes in elastin fibers, 8 weeks after the biodegradable polymer dispersion according to Example 1 and the polymers according to Comparative Examples 1 and 2 were injected into a B6 mouse, regarding Experimental Example 3 of the present invention.
FIG. 13 is microscope images for confirming changes in new/mature fibers, 8 weeks after the biodegradable polymer dispersion according to Example 1 and the polymers according to Comparative Examples 1 and 2 were injected into a B6 mouse, regarding Experimental Example 3 of the present invention.
FIG. 14 is a graph showing changes in new/mature fibers, 8 weeks after the biodegradable polymer dispersion according to Example 1 and the polymers according to Comparative Examples 1 and 2 were injected into a B6 mouse, regarding Experimental Example 3 collagen fiber present invention.
FIG. 15 is graphs showing expression changes of Tropoelastin and EBP factors, 8 weeks after the biodegradable polymer dispersion according to Example 1 and the polymers according to Comparative Examples 1 and 2 were injected into a B6 mouse, regarding Experimental Example 3 of the present invention.

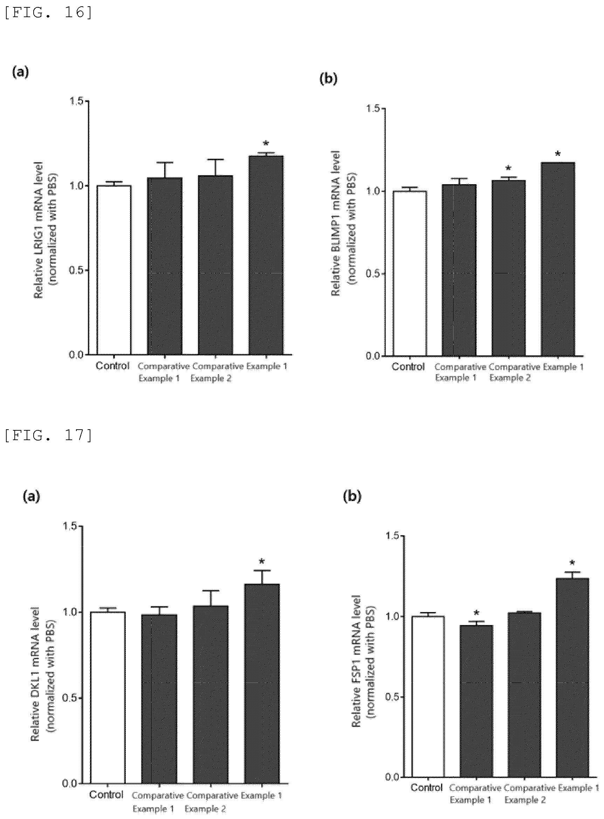

FIG.16 is graphs showing expression levels of a dermal fibroblast precursor Lrig1 and a papillary dermal fibroblast precursor Blimp1, 8 weeks after the biodegradable polymer dispersion according to Example 1 and the polymers according to Comparative Examples 1 and 2 were injected into a B6 mouse, regarding Experimental Example 3 of the present invention.
FIG. 17 is graphs showing expression levels of dermis reticular dermal/subcutaneous fibroblast precursor Dlk1 and papillary dermal fibroblasts FSP1, 8 weeks after the biodegradable polymer dispersion according to Example 1 and the polymers according to Comparative Examples 1 and 2 were injected into a B6 mouse, regarding Experimental Example 3 of the present invention.

### [Best Mode]

Hereinafter, the biodegradable polymer dispersion according to the present invention, a method for preparing the same, and a pharmaceutical composition including the same will be described in detail.

Herein, unless otherwise defined, all technical terms and scientific terms have the same meanings as those commonly understood by a person skilled in the art to which present invention pertains.

The terms used herein are only for effectively describing a certain specific example and are not intended to limit the present invention.

In addition, in describing constituent elements of the present invention, terms such as first, second, A, B, (a), and (b) may be used. These terms are used only to differentiate the constituent elements from other constituent elements, and the nature, sequence, order, or the like of the corresponding constituent elements is not limited by these terms.

In addition, unless otherwise stated in the present specification, the unit of added materials may be wt%.

In addition, the singular form used in the present specification may be intended to also include a plural form, unless otherwise indicated in the context.

In addition, the term "comprise" in the present specification is an open-ended description having a meaning equivalent to the term such as "is/are provided", "contain", "have", or "is/are characterized", and does not exclude elements, materials, or processes which are not further listed.

In the following description, description for well-known effects and configurations that may obscure the gist of the present invention unnecessarily will be omitted.

The term "lactic acid-based polymer particles" used in the present invention is used with the same meaning as "particles including a lactic acid-based polymer".

The term "PEG-PLA block copolymer" used in the present invention is used in a more specific sense than "block copolymer including a lactic acid-based polymer and polyethylene glycol".

The term "dispersion" used in the present invention refers to a "dispersion formed in a water phase" and is used in the sense of including a "biodegradable polymer dispersion".

The term "skin regeneration" used in the present invention refers to reduction, weakening, delay, or reverse of signs of skin aging or skin damage. In addition, it may include the meaning of improving cosmetic appearance or cosmetic improvement of the skin. Specifically, for example, it may include an increase in skin brightness, a decrease in pore size, wrinkle reduction, improvement of mottled complexion due to freckles, blotch, and the like, relief of sagging skin due to tissue loss and the like.

The present invention may provide a biodegradable polymer dispersion including: a lactic acid-based polymer; a block copolymer including a lactic acid-based polymer and polyethylene glycol; and a high molecular weight hyaluronic acid having a weight average molecular weight of 500,000 g/mol or more.

The lactic acid-based polymer refers to a polymer including a lactic acid in a structural unit, and the lactic acid may be L-lactic acid, D-lactic acid, or a combination thereof. A lactic acid unit may be included at 50 mol% or more, specifically 60 mol% or more, and still more specifically 70 mol% or more, with respect to 100 mol% of all monomer components forming the lactic acid-based polymer, but is not limited thereto.

The lactic acid-based polymer may include poly(lactic acid) (PLA), poly(lactic-co-glycolic acid) (PLGA), and the like, and specifically, may be poly(lactic acid) (PLA).

The lactic acid-based polymer may have a weight average molecular weight of 10,000 to 1,000,000 g/mol, specifically 13,000 to 500,000 g/mol, and more specifically 15,000 to 250,000 g/mol.

The block copolymer including the lactic acid-based polymer and polyethylene glycol may refer to a polymer including the lactic acid-based polymer and a polyethylene glycol unit. Specifically, it may be a double block copolymer of the lactic acid-based polymer and polyethylene glycol (PEG-PLA block copolymer). Herein, since the lactic acid-based polymer is hydrophobic, and the polyethylene glycol is hydrophilic, the block copolymer may be amphipathic.

The lactic acid-based polymer in the block copolymer including the lactic acid-based polymer and polyethylene glycol may be a polymer of a monomer selected from the group consisting of L-lactic acid, D-lactic acid, and L,D-lactic acid, and may have a weight average molecular weight of 1,000 to 40,000 g/mol, specifically 1,500 to 30,000 g/mol. The block copolymer may have the weight average molecular weight of 2,000 to 60,000 g/mol, and the polyethylene glycol in the block copolymer may have the weight average molecular weight of 1,000 to 20,000 g/mol, specifically 3,000 to 15,000 g/mol, but is not limited thereto. A weight ratio between a lactic acid-based polymer block and a polyethylene glycol block in the block copolymer may be 95:5 to 50:50, specifically 90:10 to 70:30, and more specifically 90:10 to 80:20.

The lactic acid-based polymer and the block copolymer including the lactic acid-based polymer and polyethylene glycol may be included at a weight ratio of 10:1 to 1:1. Since the lactic acid-based polymer and the block copolymer are included together in the weight range, the particle size controllability and dispersion stability of the lactic acid-based polymer particles of the present invention may be improved, and the miscibility with a hyaluronic acid mixture described later is improved, which is further preferred. In addition, since the weight ratio between the lactic acid-based polymer and the block copolymer is adjusted, the size and the shape of the dispersion formed in a water phase may be adjusted.

More specifically, when the weight ratio between the lactic acid-based polymer and the block copolymer is 6:1 to 2:1, the dispersion may be prepared into fine particles having a size of 0.01 to 4 µm, 0.1 to 1 µm, or 150 to 500 nm, and the fine particles may have a spherical shape in which the lactic acid-based polymer is densely filled.

The dispersion formed in a water phase by including the lactic acid-based polymer and the block copolymer including the lactic acid-based polymer and polyethylene glycol may be spherical or oval particles, and the particles may have an average particle size of 0.01 to 30 µm, specifically 0.1 to 20 µm.

The dispersion using the lactic acid-based polymer alone may have an average particle size allowing atomization depending on a preparation process, but has high polydispersity and poor dispersion reproducibility. However, since the biodegradable polymer dispersion according to the present invention includes the block copolymer including the lactic acid-based polymer and polyethylene glycol, it shows very high reproducibility depending on the preparation method, allows atomization without application of high energy, and has high particle dispersibility.

The hyaluronic acid may be in a mixed form which further includes one or more of a hyaluronic acid oligomer and an ammonium-substituted hyaluronic acid in addition to a high molecular weight hyaluronic acid having a weight average molecular weight of 500,000 g/mol or more. Specifically, for example, it may be a mixture of a high molecular weight hyaluronic acid and a hyaluronic acid oligomer, a mixture of a high molecular weight hyaluronic acid and an ammonium-substituted hyaluronic acid, or a mixture of the three hyaluronic acids.

In the hyaluronic acid mixture, the high molecular weight hyaluronic acid may have a weight average molecular weight of 500,000 g/mol to 3,000,000 g/mol, specifically 1,200,000 g/mol to 2,200,000 g/mol. The high molecular weight hyaluronic acid may be included at 50 to 90 wt%, specifically 65 to 85 wt%, with respect to the total weight of the hyaluronic acid mixture, but is not limited thereto.

In the hyaluronic acid mixture, the ammonium-substituted hyaluronic acid may refer to a hyaluronic acid in which a part or all of hydrogen atoms of a hydroxyl group of the hyaluronic acid is substituted by a group having a quaternary ammonium cation group, may have a weight average molecular weight of 300,000 g/mol to 1,000,000 g/mol, specifically 500,000 g/mol to 800,000 g/mol, and may be included at 1 to 10 wt%, specifically 3 to 8 wt% with respect to the total weight of the hyaluronic acid mixture, but is not limited thereto.

In the hyaluronic acid mixture, the hyaluronic acid oligomer may mean a hydrolyzed state of hyaluronic acid. It has a weight average molecular weight of 800 g/mol or more and less than 8,000 g/mol, may refer to a low molecular weight hyaluronic acid of less than 6,000 g/mol, and more specifically, may have a weight average molecular weight of 1,000 to 5,000 g/mol or 2,000 to 4,000 g/mol. The hyaluronic acid oligomer may be included at 5 to 50 wt%, specifically 5 to 20 wt%, or 10 to 20 wt%, with respect to the total weight of the hyaluronic acid mixture, and may show an appropriate density for skin penetration.

A weight ratio between the hyaluronic acid mixture and the lactic acid-based polymer may be 1.5:1 to 20:1, specifically 2:1 to 10:1, more specifically 2:1 to 8:1. When the weight ratio falls within the above range, the dispersion can be effectively reconstituted in a resuspension process after a freeze-drying treatment, when they are prepared into the biodegradable polymer dispersion.

Since the mixture of three different hyaluronic acids as described above is used, dispersion stability of lactic acid-based polymer particles of the biodegradable polymer dispersion described later may be further improved. In particular, since the hyaluronic acid mixture is bound to the lactic acid-based polymer particles, an interaction with a skin barrier layer may be significantly improved. Since a usual lactic acid-based polymer has high crystallinity and low interaction with a lipid layer forming the skin barrier layer, it is known to have significantly low adsorption or penetration properties to the skin barrier layer. However, when the hyaluronic acid mixture is mixed with the lactic acid-based polymer particles and hydrates the surface of the lactic acid-based polymer particles, the interaction of the lactic acid-based polymer particles with the skin barrier layer is significantly improved so that the lactic acid-based polymer particles may be strongly adsorbed on or penetrate the skin barrier layer. Accordingly, when the biodegradable polymer dispersion is applied to the skin, a skin penetration effect is excellent, thereby further improving physiological activity, such as improving elasticity of skin cells, suppressing aging, and promoting skin cell growth.

In the biodegradable polymer dispersion according to a preferred exemplary embodiment of the present invention, lactic acid-based polymer particles may be dispersed in a continuous phase including the hyaluronic acid mixture, and a polylactic acid-polyethylene glycol block copolymer (PLA-PEG diblock copolymer) may be positioned on the surface of the lactic acid-based polymer particles. More specifically, in the block copolymer, a hydrophobic polylactic acid portion may be positioned to be directed toward the lactic acid-based polymer particles and hydrophilic polyethylene glycol may be positioned to be directed toward the continuous phase. Herein, since the hydrophobic portion of the block copolymer may be positioned more densely on the surface of the particles by the hyaluronic acid mixture of three different hyaluronic acids forming the continuous phase, and excellent dispersion stability may be implemented by polyethylene glycol which is a hydrophilic portion, a large amount of lactic acid-based polymer particles as a dispersion phase may be accommodated in the continuous phase, which is thus more preferred. Accordingly, the biodegradable polymer dispersion according to the present invention may include the active ingredient in a continuous phase and in a dispersion phase independently of each other, which is thus more preferred.

Hereinafter, a method for preparing a biodegradable polymer dispersion according to the present invention will be described in detail.

The method for preparing a biodegradable polymer dispersion according to the present invention may include: dissolving a block copolymer including a lactic acid-based polymer and polyethylene glycol and a lactic acid-based polymer in a first organic solvent to prepare a biodegradable polymer solution; mixing a hyaluronic acid and water to prepare a hyaluronic acid aqueous solution; mixing the hyaluronic acid aqueous solution with the biodegradable polymer solution to prepare a first dispersion liquid; and removing the first organic solvent from the first dispersion liquid to obtain a first emulsion.

In addition, the method may further include: redispersing the first emulsion in a second organic solvent to prepare a second dispersion liquid; and removing the second organic solvent from the second dispersion liquid, thereby having an effect of further improving dispersion stability upon resuspension.

The step of preparing a biodegradable polymer solution may be performed by dissolving the lactic acid-based polymer and the block copolymer described above in an organic solvent, and the organic solvent may be any one or a mixture of two or more selected from the group consisting of acetone, ethanol, methylene chloride, chloroform, ethylacetate, tetrahydrofuran, N,N-dimethylformamide, and N,N-dimethylacetamide, and preferably acetone.

The organic solvent may be included at 70 wt% to 95 wt% with respect to the total weight of the biodegradable polymer solution, but is not limited thereto.

In the hyaluronic acid aqueous solution, one hyaluronic acid, a mixture of two hyaluronic acids of the high molecular weight hyaluronic acid and the low molecular hyaluronic acid, or a mixture of three hyaluronic acids of the ammonium-substituted hyaluronic acid with the two hyaluronic acids is mixed in water, so that water may be included at 95 wt% to 99.9 wt% with respect to the total aqueous solution, but the present invention is not limited thereto. Herein, a mixing ratio of the two or three hyaluronic acids is as described above.

The step of preparing the first dispersion liquid may be mixing the biodegradable polymer solution and the hyaluronic acid aqueous solution at a volume ratio of 1:1 to 1:3. Herein, in the first dispersion liquid, the aqueous solution forms a continuous phase and the organic solvent is extracted into the continuous phase, so that the phase separated polymer may form a dispersion phase. Specifically, in the dispersion phase, the block copolymer including the lactic acid-based polymer and polyethylene glycol may be positioned on the surface of the dispersed lactic acid-based polymer particles.

The step of removing the organic solvent is not limited, but specifically, may use evaporation, and the evaporation conditions may be performed at 20 to 130°C for 1 hour to 48 hours, but is not limited thereto, and the evaporation conditions may be adjusted depending on the type of solvent to be used. In addition, the evaporation may be performed under reduced pressure.

When the first emulsion is obtained by removing the first organic solvent, it may be redispersed in the second organic solvent to prepare a second dispersion liquid. Herein, as a preferred exemplary embodiment of the present invention, a step of freeze-drying the obtained first emulsion is further included to secure the first dispersion in a solid state, and the first dispersion may be redispersed in the second organic solvent to prepare the second dispersion liquid. In this case, resuspension may be performed more stably.

The biodegradable polymer dispersion of the present invention prepared by the steps described above may be in a form in which the lactic acid-based polymer particles are dispersed in a continuous phase including the hyaluronic acid mixture, and the lactic acid-based polymer particles have the block copolymer including the lactic acid-based polymer and polyethylene glycol positioned on the surface.

Since the biodegradable polymer dispersion according to an exemplary embodiment of the present invention has excellent physiological activity characteristics, biocompatibility, and high supporting characteristics to include a large amount of active ingredients on its own, it may be applied to various fields, and specifically, may be used as a cosmetic composition, a composition for external skin application, a pharmaceutical composition, or the like including the biodegradable polymer dispersion.

As an example, the pharmaceutical composition for skin regeneration including the biodegradable polymer dispersion may be provided. Specifically, the present invention may provide a local skin regeneration effect on a soft tissue by deriving collagen synthesis. More specifically, when it is injected around wounds on any part of the body such as face, neck, chest, buttocks, arms, armpits, hands, legs, or feet, wound healing is promoted, scar formation is inhibited, and a scar treatment effect on the wound site may be provided.

Herein, the "soft tissue" refers to a tissue which connects, supports, or wraps other structures and organs of the body other than bones. Specifically, an injectable soft tissue may include tendons, ligaments, fascia, skin, dermis, fibrous tissue, fat, synovium, muscles, nerves and blood vessels.

As an example, a cosmetic composition for improving wrinkles including the biodegradable polymer dispersion may be provided. The composition including the biodegradable polymer dispersion according to the present invention may derive collagen production by activating fibroblasts or fibroblast precursor. The fibroblast stimulates production of structural protein such as collagen and elastin, and the collagen produced by the fibroblast may form a fibrous connective tissue which maintains skin elasticity. By injecting the composition according to the present invention into an area where skin damage occurs, scar and wrinkle improvement effect may be provided.

As an example, a pharmaceutical composition for skin regeneration or wound healing including the biodegradable polymer dispersion may be provided. Herein, the pharmaceutical composition may include a pharmaceutically acceptable carrier, if necessary, and may be formulated into a formulation for transdermal administration such as a liquid, a suspension, an emulsion, a lotion, and an ointment according to a common method.

The pharmaceutically acceptable carrier includes an aqueous diluent or solvent such as a phosphate buffered saline, purified water, and sterile water, and may include a non-aqueous diluent or a solvent such as propylene glycol and olive oil.

The pharmaceutical composition may vary depending on patient's condition, a degree of disease, a drug form, and the route and period of administration, and may be appropriately selected by a person skilled in the art.

In addition, a first embodiment in which a hydrophilic active ingredient is included in the hyaluronic acid mixture which is a hydrophilic continuous phase, a second embodiment in which a hydrophobic active ingredient is included inside the hydrophobic lactic acid-based polymer, and a third embodiment in which an active ingredient is included in each of the hyaluronic acid mixture which is a hydrophilic continuous phase and the hydrophobic lactic acid-based polymer are allowed.

The active ingredient may be used without limitation as long as it is a known active component having physiological activity in the skin or other tissues, and a common additive which is not harmful to the human body, such as additional flavorings, vitamins, stabilizers, and antioxidants, may be further included within a range which does not impair the physical properties of the biodegradable polymer dispersion of the present invention.

The biodegradable polymer dispersion of the present invention or the composition including the biodegradable polymer dispersion may be applied as known formulations for internal use and external use, and preferably, may be applied as a formulation for internal use such as cream, ointment, lotion, and gel.

In addition, the present invention may provide a skin regeneration method or a skin wound healing method including administering the pharmaceutical composition including the biodegradable polymer dispersion described above.

The composition including the biodegradable polymer dispersion or the biodegradable polymer dispersion may be directly applied on the skin, and when a plasma treatment is performed on the applied area, the biodegradable polymer dispersion may penetrate into the skin, and as the absorption into the skin is promoted, physiological activity characteristics may be accelerated, and thus, an effective skin improvement is allowed within a short time, and it may help the treatment by skin regeneration, and thus, may be applied to skin improvement.

In addition, when an additional active ingredient is included in the biodegradable polymer dispersion, diffusion of the active ingredient into the deep skin layer is promoted by the plasma treatment, thereby further maximizing the skin improvement or the treatment effect, which is thus preferred.

The biodegradable polymer dispersion according to an exemplary embodiment of the present invention having the characteristics described above and the composition including the same are provided together with a plasma treatment device and may be used as a preferred skin care system.

Hereinafter, the present invention will be described in more detail with reference to the examples and the comparative examples. However, the following examples and comparative examples are provided only to describe the present disclosure in more detail, and do not limit the present disclosure in any way.

### [Example 1]

A biodegradable polymer solution in which 0.25 g of polylactic acid (EVONIK, RESOMER^{®} R 202 S) and 0.125 g of PEG-PLA block copolymer (EBONIK, RESOMER 100 DL mPEG5000) were mixed in 25 mL of acetone was prepared.

9.0 g of hyaluronic acid (molecular weight: 1,200,000 g/mol) and 1.0 g of sodium hyaluronate (molecular weight: 5,000 g/mol) which was hydrolyzed into a hyaluronic acid oligomer were mixed in 1,000 mL of distilled water to prepare an aqueous solution.

Thereafter, 25 mL of the polymer solution prepared above was slowly mixed with 50 mL of an aqueous solution under stirring, and acetone in the solution after mixing was evaporated under reduced pressure at room temperature to obtain an emulsion state, which was then freeze-dried to obtain a dispersion. The dispersion was redispersed in an organic solvent, and the organic solvent was evaporated under reduced pressure to prepare a biodegradable polymer dispersion.

### [Example 2]

A biodegradable polymer dispersion was prepared in the same manner as in Example 1, except that 9.0 g of a hyaluronic acid (molecular weight: 1,200,000 g/mol) and 1.0 g of hydroxypropylammonium hyaluronic acid (molecular weight: 500,000 g/mol) as an ammonium-substituted hyaluronic acid were mixed in 1,000 mL of distilled water to prepare a hyaluronic acid aqueous solution.

### [Example 3]

A biodegradable polymer dispersion was prepared in the same manner as in Example 1, except that 10.0 g of a hyaluronic acid (molecular weight: 1,200,000 g/mol) was mixed in 1,000 mL of distilled water.

### [Example 4]

A biodegradable polymer dispersion was prepared in the same manner as in Example 1, except that 0.25 g of polylactate-co-glycolate (EVONIK, RESOMER RG 752 S) was used instead of the polylactic acid, and 0.125 g of a PEG-PLA copolymer was used.

### [Comparative Example 1]

A hydrogel filler prototype of a hyaluronic acid crosslinked product which was prepared using 1,4-butanediol diglycidylether (BDDE) as a crosslinking agent was prepared. The filler was a product including small grains in a particulate form.

### [Comparative Example 2]

A biodegradable polymer dispersion was prepared by dissolving 2.5 g of PLA in 20 mL of ethyl acetate to prepare a biodegradable polymer solution, dispersing the solution in 100 mL of water containing 5 g of Tween 80 (polyoxyethylene sorbitan monooleate), and then evaporating the solvent.

### [Comparative Example 3]

A biodegradable polymer dispersion was prepared in the same manner as in Example 1, except that 0.125 g of a polylactic acid was used, and 2.125 g of a hyaluronic acid (molecular weight: 1,200,000 g/mol), 0.125 g of a hydroxypropyltriammonium hyaluronic acid (molecular weight: 500,000 g/mol) as the ammonium-substituted hyaluronic acid, and 0.25 g of sodium hyaluronate which was hydrolyzed into a hyaluronic acid oligomer (molecular weight: 5,000 g/mol) were mixed in 1,000 mL of distilled water to prepare an aqueous solution.

### [Comparative Example 4]

A biodegradable polymer dispersion was prepared in the same manner as in Example 1, except that 2.125 g of a hyaluronic acid (molecular weight: 1,200,000 g/mol), 0.125 g of a hydroxypropyltriammonium hyaluronic acid (molecular weight: 500,000 g/mol) as the ammonium-substituted hyaluronic acid, and 0.25 g of sodium hyaluronate which was hydrolyzed into a hyaluronic acid oligomer (molecular weight: 5,000 g/mol) were mixed in 1,000 mL of distilled water to prepare an aqueous solution.

### [Comparative Example 5]

A biodegradable polymer dispersion was prepared in the same manner as in Example 1, except that 0.125 g of a polylactic acid was used, 0.7 g of a hyaluronic acid (molecular weight: 1,200,000 g/mol), 0.04 g of a hydroxypropyltriammonium hyaluronic acid (molecular weight: 500,000 g/mol) as the ammonium-substituted hyaluronic acid, and 0.08 g of sodium hyaluronate which was hydrolyzed into a hyaluronic acid oligomer (molecular weight: 5,000 g/mol) were mixed in 1,000 mL of distilled water to prepare an aqueous solution.

The characteristics of the examples and the comparative example were evaluated as follows.

### [Experimental Example 1] Evaluation of production and redispersibility of biodegradable polymer dispersion

The biodegradable polymer dispersions prepared in Examples 1 to 4 and Comparative Examples 3 to 5 were resuspended in distilled water and an emulsion state was observed, and the particle size of particles dispersed in the biodegradable polymer dispersions was measured and the nanoparticle percentage is shown in Table 1 below. At this time, the nanoparticles were based on those having an average particle diameter of 100 to 1000 nm.

Examples 1 to 4 had a nanoparticle portion of at least 20% and it was confirmed that the nanoparticles were successfully formed, but the biodegradable polymer dispersions according to Comparative Examples 3 to 5 showed a nanoparticle portion of less than 5% and it was found the nanoparticles were not formed well. It was confirmed that the examples according to the present invention had excellent nanoparticle formation as compared with Comparative Examples 3 to 5.

**[Table 1]**

| | Example | | | | Comparative Example | | |
|---|---|---|---|---|---|---|---|
| | 1 | 2 | 3 | 4 | 3 | 4 | 5 |
| Nanoparticles (%) | 25 | 21 | 22 | 20 | 5 | 2 | 3 |

### [Experimental Example 2] Evaluation of efficacy (in vitro) of biodegradable polymer dispersion for stem cells

Human adipose derived mesenchymal stem cells (hAD-MSC) were cultured in a Human MSC growth medium (CEFOgro^{™}) of 60 cm². 120 µl of the samples prepared in Example 1 and Comparative Examples 1 and 2 and PBS were treated for 48 hours in cultured hAD-MSC. RNAiso Plus (Cat. RNA was extracted according to a predetermined protocol using RNAiso Plus (Cat. 9109) reagent (Takara, Japan) from hAD-MSC treated with the sample. The extracted RNA was quantified and cDNA was synthesized. cDNA was synthesized according to the predetermined protocol using PrimeScript^{™} 1st strand cDNA Synthesis Kit (Cat. 6110A) (Takara, Japan). In order to confirm the mRNA value using the synthesized cDNA, 0.2 µM of target gene primer and 200 ng of cDNA mixture were mixed with SYBR^{™} green and a quantitative gene amplification experiment was performed.

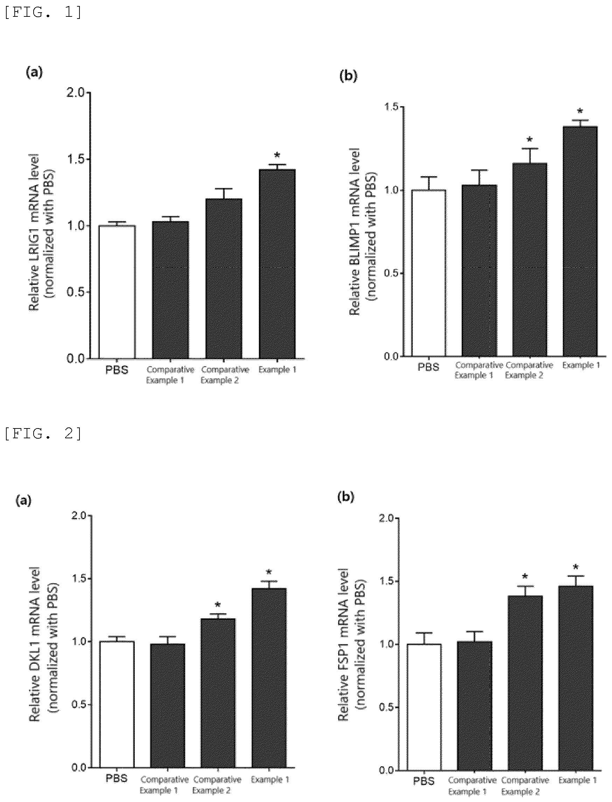

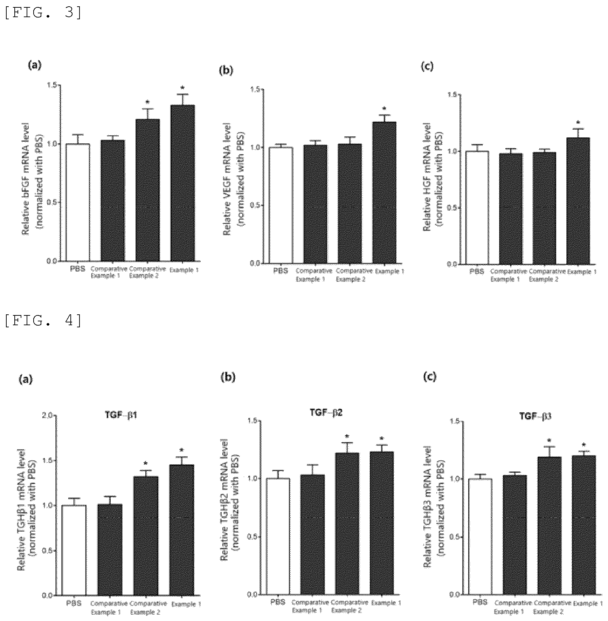

Thereafter, secretion degrees of growth factor and dermal fibroblast precursor in adipose-derived stem cells of the biodegradable polymer dispersions according to Example 1 and Comparative Examples 1 and 2 were measured, and the results are shown in FIGS. 1 to 4.

### [Experimental Example 3] Evaluation of elasticity improvement effect (in vivo) of biodegradable polymer dispersion

Five 12-month-old male C57BL6J(B6) mice were set as one group, 100 µ l of each of the samples of Example 1 and Comparative Examples 1 and 2 was injected into 5 points of the skin using a 27G screw needle, and after 8 weeks, a mouse included in the corresponding group was eliminated.

### 1. Measurement of moisture and elasticity

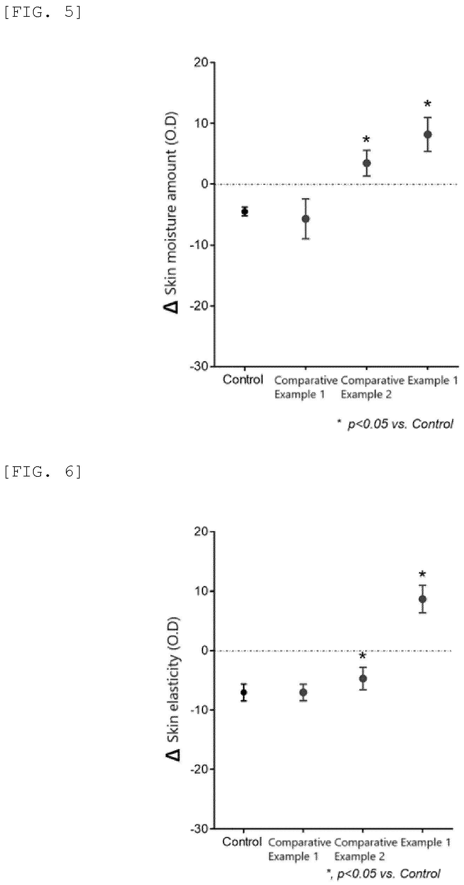

Moisture and elasticity were measured at two time points before treating the sample and before collecting the tissue after 8 weeks. 5 values were measured by skin analysis equipment in a state of smooth skin by removing hair, and similar 3 values among them were read and shown in FIGS. 5 and 6.

### 2. Tissue collection and examination

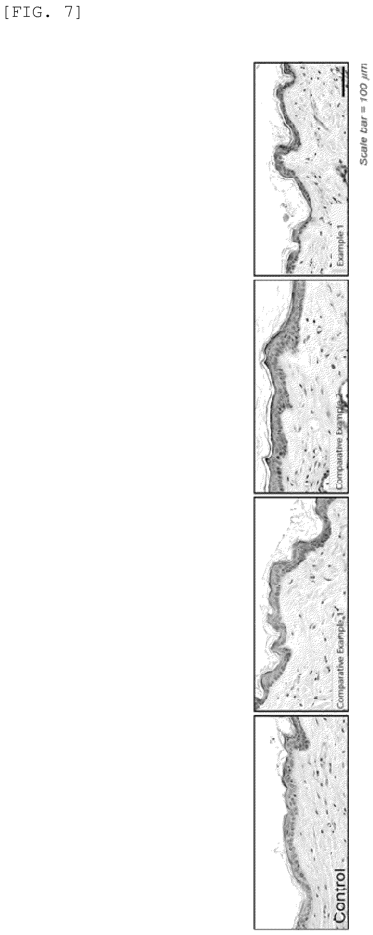

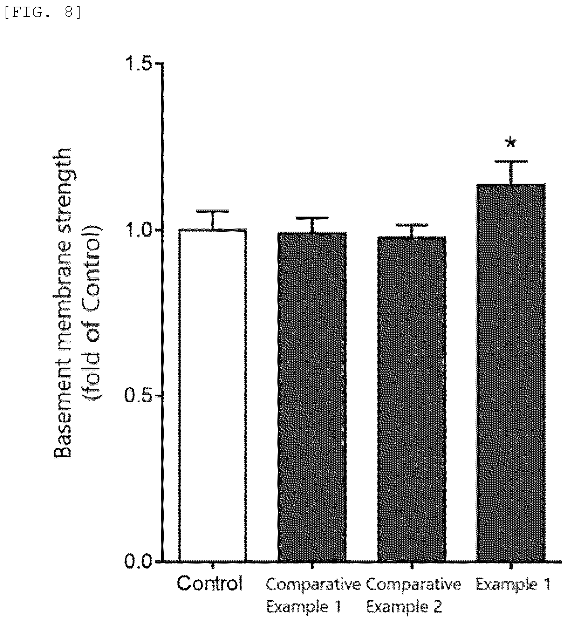

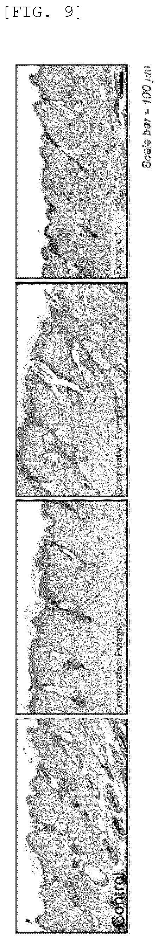

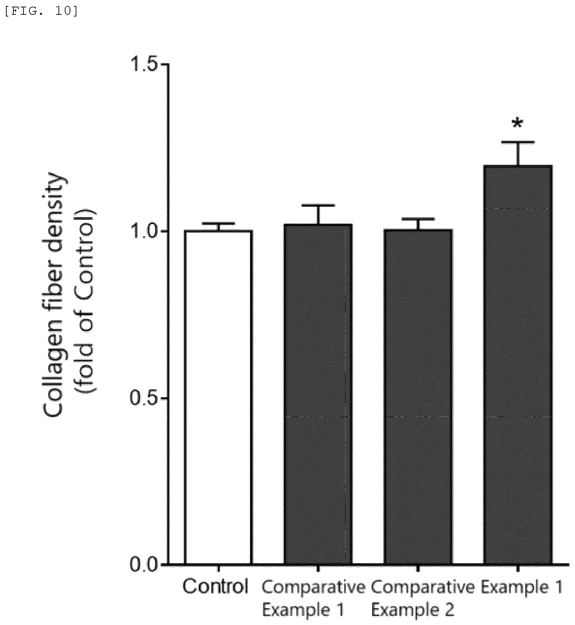

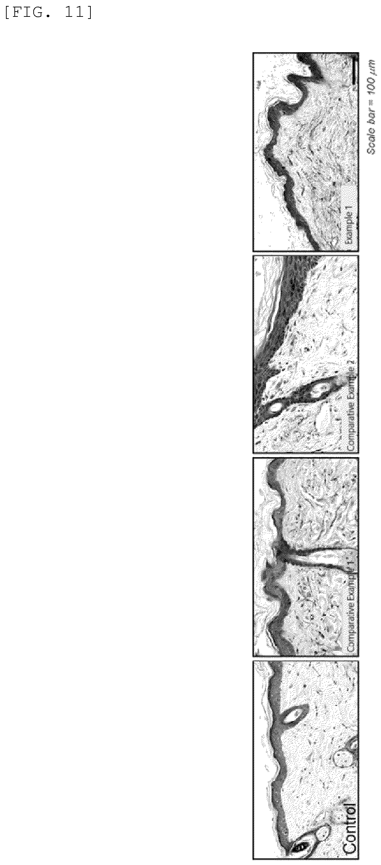

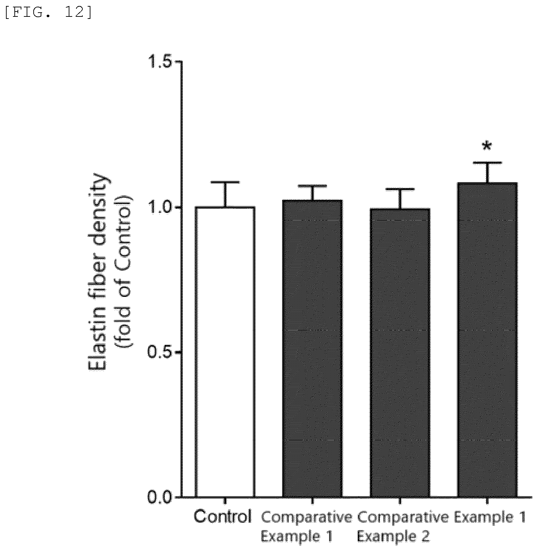

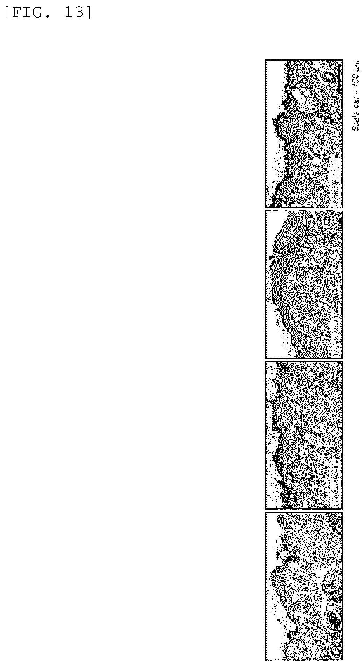

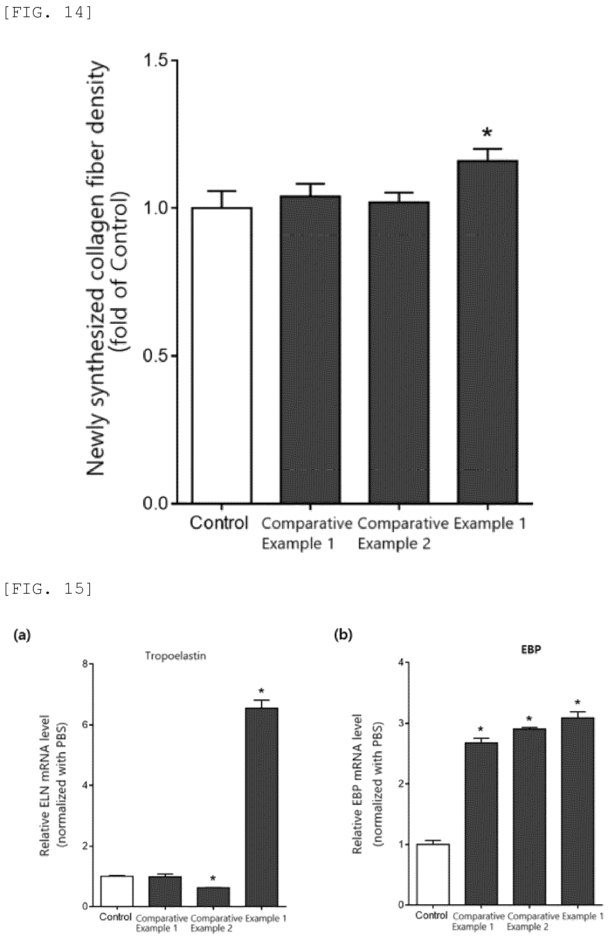

After removing hair from the mouse's back, the tissue surrounding the area treated with the sample was cut wide, and it was observed with the naked eye whether the sample remained in the skin. Thereafter, the tissue was cut into several equal parts, which were placed in a clean tube, frozen using liquid nitrogen, and stored in an ultra-low temperature freezer and fixed. The fixed skin tissue was prepared into a paraffin block using a tissue processor and a tissue embedding machine and cut into a section of 7 µm using a cutter, and tissue examination was performed.
(1) Periodic Acid Schiff dyeing kit (Cat. SSK5020, BBC biochemical) was used to confirm a change in a basement membrane through Periodic acid-Schiff (PAS) dyeing. The results are shown in FIGS. 7 and 8. The basement membrane was present between epidermis and dermis, and a PAS positive signal was expressed as a deep red color. In Example 1, the PAS positive signal was statistically significantly increased as compared with an aged mouse (control), but in Comparative Examples 1 and 2, there was no change.
(2) Trichrome dyeing kit (Modified Masson's) (Cat. TRM-IFU, Scy Tek Laboratories) was used to confirm a change in collagenous fibers through Masson trichrome (MT) dyeing. The results are shown in FIGS. 9 and 10. A signal expressed in blue in the dermis of the skin tissue after MT dyeing was collagenous fibers. In Example 1, the MT positive signal was statistically significantly increased as compared with an aged mouse (control), but in Comparative Examples 1 and 2, there was no change.
(3) Elastic dyeing kit (Modified Verhoff's) (Cat. ETS-1-IFU, Scy Tek Laboratories) was used to confirm a change in elastin fibers through Verhoeff dyeing. The results are shown in FIGS. 11 and 12. A signal expressed in blue in the dermis of the skin tissue after Verhoeff dyeing was elastic fibers. In Example 1, the Verhoeff positive signal was statistically significantly increased as compared with an aged mouse (control), but in Comparative Examples 1 and 2, there was no change.
(4) Herovici dyeing kit (Cat. HSK-IFU, Scy Tek Laboratories) was used to confirm a change in new/mature collagenous fibers through Herovici dyeing. The results are shown in FIGS. 13 and 14. A signal expressed in blue in the dermis layer of the skin tissue after Herovici dyeing was new/mature collagenous fibers. In Example 1, the Verhoeff positive signal was statistically significantly increased as compared with an aged mouse (control), but in Comparative Examples 1 and 2, there was no change.
(5) RNA was extracted from 100 mg of the tissue stored in an ultra-low temperature refrigerator using a RNAiso Plus (Cat. 9109, Takara) reagent. The extracted RNA was quantified and cDNA was synthesized using PrimeScript^{™} 1st strand cDNA Synthesis Kit (Cat. 6110A, Takara). In order to confirm the mRNA value using the synthesized cDNA, 0.2 µM of target gene primer and 200 ng of cDNA mixture were mixed with SYBR^{™} green and a quantitative gene amplification experiment was performed. Data are expressed as mean ± standard error of the mean (SEM) of 3 independent experiments. (*, P <0.05)

Hereinabove, although the present disclosure has been described by specific matters, limited exemplary embodiments, and drawings, they have been provided solely to aid the overall understanding of the present disclosure, and the present disclosure is not limited to the exemplary embodiments, and various modifications and changes may be made by those skilled in the art to which the present disclosure pertains from the description.

Therefore, the spirit of the present invention should not be limited to the above-described exemplary embodiments, and the following claims as well as all modifications equal or equivalent to the claims are intended to fall within the scope and spirit of the invention.

## Claims

1. A biodegradable polymer dispersion comprising: a lactic acid-based polymer; a block copolymer including a lactic acid-based polymer and polyethylene glycol; and a high molecular weight hyaluronic acid having a weight average molecular weight of 500,000 g/mol or more.

2. The biodegradable polymer dispersion of claim 1, wherein a weight ratio between the hyaluronic acid and the lactic acid-based polymer is 1.5:1 to 5:1.

3. The biodegradable polymer dispersion of claim 1, wherein the hyaluronic acid further includes one or more of a hyaluronic acid oligomer having a weight average molecular weight of less than 6,000 g/mol and an ammonium-substituted hyaluronic acid.

4. The biodegradable polymer dispersion of claim 3, wherein the hyaluronic acid oligomer is included at 5 to 20 wt% with respect to the total weight of the hyaluronic acid.

5. The biodegradable polymer dispersion of claim 1, wherein the biodegradable polymer dispersion comprises spherical particles having an average particle size of 0.01 to 30 µm.

6. The biodegradable polymer dispersion of claim 1, wherein the lactic acid-based polymer has a weight average molecular weight of 10,000 to 1,000,000 g/mol.

7. The biodegradable polymer dispersion of claim 1, wherein the block copolymer including the lactic acid-based polymer and polyethylene glycol has a weight average molecular weight of 2,000 to 60,000 g/mol.

8. The biodegradable polymer dispersion of claim 1, wherein a weight ratio between the lactic acid-based polymer and the block copolymer including the lactic acid-based polymer and polyethylene glycol is 10:1 to 1:1.

9. The biodegradable polymer dispersion of claim 1, wherein the block copolymer including the lactic acid-based polymer and polyethylene glycol is positioned on the surface of particles including the lactic acid-based polymer.

10. A pharmaceutical composition comprising the biodegradable polymer dispersion of claim 1.

11. A method for preparing the biodegradable polymer dispersion of claim 1, the method comprising:
dissolving a block copolymer including a lactic acid-based polymer and polyethylene glycol and a lactic acid-based polymer in a first organic solvent to prepare a biodegradable polymer solution;
mixing a high molecular weight hyaluronic acid having a weight average molecular weight of 500,000 g/mol or more and water to prepare a hyaluronic acid aqueous solution;
mixing the hyaluronic acid aqueous solution with the biodegradable polymer solution to prepare a first dispersion liquid; and
removing the first organic solvent from the first dispersion liquid to obtain a first emulsion.

12. The method for preparing the biodegradable polymer dispersion of claim 11, wherein the organic solvent is one or a mixture of two or more selected from the group consisting of acetone, ethanol, methylene chloride, chloroform, ethyl acetate, tetrahydrofuran, N,N-dimethylformamide, and N,N-dimethylacetamide.

13. The method for preparing the biodegradable polymer dispersion of claim 11, wherein the hyaluronic acid aqueous solution is further mixed with one or more of a hyaluronic acid oligomer having a weight average molecular weight of less than 6,000 g/mol and an ammonium-substituted hyaluronic acid.

14. The method for preparing the biodegradable polymer dispersion of claim 11, further comprising:
redispersing the first emulsion in a second organic solvent to prepare a second dispersion liquid; and
removing the second organic solvent from the second dispersion liquid.

15. The method for preparing the biodegradable polymer dispersion of claim 14, wherein a ratio of nanoparticles having an average particle diameter of 100 to 1000 nm in the biodegradable polymer dispersion is 20% or more.
